# EUROPÄISCHE PATENTANMELDUNG

(11) **EP 0 718 270 A2**
(43) Veröffentlichungstag der Anmeldung: **26.06.1996**
(21) Anmeldenummer: 95120313.2
(22) Anmeldetag: 21.12.1995
(51) Int. Cl.: C07C 43/13, C07C 43/04, C07C 41/06

(54) **Verfahren zur Herstellung von Polyolalkylethern**

(30) Priorität: 21.12.1994 DE 4445635
(71) Anmelder: Wessendorf, Richard, Dr., D-45259 Essen (DE)
(72) Erfinder: Wessendorf, R. Dr., D-45259 Essen (DE); Graf, Wilfried, D-46282 Dorsten (DE)

(57) **Zusammenfassung**

Verfahren zur Herstellung von Polyolalkylethern, bei dem man Polyhydroxyverbindungen, ausgewählt aus der Gruppe
a) Alkylenglycole,
b) Glycerin,
d) Trimethylolpropan,
d) Pentaerythrit
in Gegenwart von sauren Katalysatoren bei Temperaturen von 50 bis 120°C und Drücken von 5 bis 30 bar mit Olefinen der Formel (I) 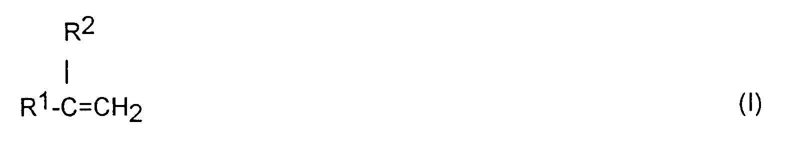
umsetzt, in der R¹ für einen linearen oder verzweigten Alkylrest mit 1 bis 4 Kohlenstoffatomen und R² für Wasserstoff oder ebenfalls für einen Alkylrest mit 1 bis 4 Kohlenstoffatomen steht. Zur Erzielung eines hohen Umsatzes in kurzer Reaktionszeit wird die Reaktion in der Flüssigphase in Gegenwart eines Lösungsmittel durchgeführt.

## Beschreibung

Die Erfindung betrifft ein Verfahren zur Herstellung von Polyolalkylethern, bei dem man Polyhydroxyverbindungen in Gegenwart von sauren Katalysatoren bei Temperaturen von 50 bis 120 °C und bei Drücken von 5 bis 30 bar mit Olefinen umsetzt, sowie die Verwendung der so hergestellten Polyolalkylether.

In der WO 94/01389 wird ein Verfahren der eingangs genannten Art zur Herstellung von Polyolalkylethern offenbart. Bezüglich der apparativen Ausgestaltung des Verfahrens ist dem Dokument zu entnehmen, daß 1-Liter-Autoklaven benutzt wurden. Die Reaktionszeit wird mit 10 bis 25 Std. angegeben. In der Beschreibung findet sich kein Hinweis auf den Einsatz von Lösungsmitteln und sich daraus ergebenden Vorteilen.

Bei der Durchführung von Autoklavversuchen, wie sie z. B. in der genannten WO 94/01389 beschrieben werden, ist zu beobachten, daß für das Gelingen der Veretherung bzw. für einen befriedigenden Umsatz innerhalb einer akzeptablen Zeit bei Verwendung eines festen Katalysators eine intensive Rührung von ca. 1500 Umdrehungen/min. erforderlich ist. Hierbei wird der feste Katalysator, z. B. Ionenaustauscherharz oder Zeolith, durch mechanische Einwirkung weitgehend beschädigt oder zerstört. Im kontinuierlich arbeitenden Rohrreaktor reicht die Strömung für eine innige Durchmischung der ineinander nicht löslichen Reaktionspartner nicht aus, um zu befriedigenden Umsätzen zu gelangen.

Aufgabe der Erfindung ist es, ein verbessertes Verfahren zur Verfügung zu stellen, bei dem innerhalb einer relativ kurzen Reaktionszeit ein hoher Umsatz erzielt wird, ohne daß im Falle des Einsatzes fester Katalysatoren diese zerstört werden.

Die Aufgabe wurde gelöst durch ein Verfahren gemäß Anspruch 1. Bevorzugte Ausführungsformen des Verfahrens sind Gegenstand der Unteransprüche.

Das erfindungsgemäße Verfahren ist dadurch gekennzeichnet, daß man die Reaktion in der Flüssigphase in Gegenwart eines Lösungsmittels durchführt. Das Lösungsmittel hat die Aufgabe, den Kontakt der Reaktionspartner mit dem Katalysator bzw. mit den aktiven Zentren des Katalysators besser und schneller zu ermöglichen. Dies ist der Fall, wenn die Reaktanten im Lösungsmittel löslich sind. Dadurch findet eine Reduzierung der an der Reaktion beteiligten Phasen statt. Geeignete Lösungsmittel sind z. B. niedere Alkohole und auch niedere Ketone, insbesondere solche mit 3 bis 9 C-Atomen, vorzugsweise Aceton. Von den niederen Alkoholen sind die mit 1 bis 4 C-Atomen besonders geeignet. Mit Vorteil können insbesondere Methanol und tert.-Butylalkohol als Lösungsmittel eingesetzt werden.

Unter den als Edukte geeigneten Polyhydroxyverbindungen sei insbesondere das Glycerin aufgrund seiner Herkunft als nachwachsender Rohstoff einerseits sowie aufgrund der hervorragenden Eignung seiner Veretherungsprodukte als Oktanzahlverbesserer in Ottokraftstoffen andererseits genannt.

Als Alkylenglycole werden hier Ethylenglycol, Propylenglycol und deren höhere Homologe mit einem durchschnittlichen Kondensationsgrad von 2 bis 12 verstanden.

Kurzkettige Olefine, die im Sinne des erfindungsgemäßen Verfahrens mit Vorteil eingesetzt werden können, sind Propen sowie die isomeren Butene, Pentene, Hexene Heptene und Oktene. Im Hinblick auf die Eigenschaften der Polyolalkylether als Oktanzahlverbesserer ist insbesondere Isobuten als Edukt geeignet.

Das molare Einsatzverhältnis von Polyhydroxyverbindung und Olefin kann 1 : 1 bis 1 : 10 betragen. Als optimal hat sich ein Verhältnis der Reaktionspartner erwiesen, bei dem auf eine OH-Gruppe 1 bis 2 Olefin-Moleküle entfallen.

Typische Beispiele für unlösliche feste Katalysatoren sind saure Ionenaustauscher, wie Amberlyst® 15, Amberlite® XE 383 oder Dowex® 50 WX 2. Desweiteren kommen als heterogene Katalysatoren Zeolithe in Betracht, die natürlicher oder synthetischer Herkunft sein können. Bevorzugt sind synthetische Zeolithe, beispielsweise Zeolithe mit der Bezeichnung A, X, Y und L. Gegenüber Ionenaustauscherharzen führen Zeolithe zu sehr uneinheitlichen Endprodukten mit sehr vielen Nebenprodukten, weshalb erstere bevorzugt sind.

Es lassen sich auch im Reaktionsgemisch lösliche saure Katalysatoren einsetzen. Dies hat den Vorteil, daß eine weitere Phasenreduzierung erreicht wird, d. h. im wesentlichen eine Einphasenreaktion vorliegt. Der Nachteil liegt darin, daß je nach Verwendung des Produktes die Katalysatoren unter Umständen aufwendig entfernt werden müssen. Ggfs. kann man den löslichen sauren Katalysator durch Neutralisation ausällen. Nach erfolgtem Abtrennen kann der Katalysator u. U. durch Ansäuren reaktiviert und wieder eingesetzt werden. Typische Beispiele für homogene lösliche Katalysatoren sind Methansulfonsäure, p-Toluolsulfonsäure, Sulfoessigsäure, Sulfobernsteinsäure, Sulfotriazetin und Dodecylbenzolsulfonsäure.

Die Umsetzung kann unter milden Reaktionsbedingungen durchgeführt werden. Typischerweise liegen die Temperaturen bei 50 bis 120°C, vorzugsweise 70 bis 90°C und die Drücke bei 5 bis 30 bar.

Hinsichtlich der Menge an eingesetztem Lösungsmittel hat sich ein Molverhältnis von Polyhydroxyverbindungen zu Lösungsmittel im Bereich von 3 : 1 bis 1 : 5 als geeignet erwiesen. Optimal ist ein Molverhältnis von 2 : 1 bis 1 : 3.

Oftmals ist es ausreichend, nur so viel Lösungsmittel zuzusetzen, daß sich ein Teil der Edukte darin löst. Diese Ausführungsform ist insbesondere dann geeignet, wenn sichergestellt ist, daß stets soviel an Edukten nachgelöst wird wie abreagiert, ohne daß der Lösungsvorgang zum geschwindigkeitsbestimmenden Schritt der Reaktion wird. Auch in den Fällen, in denen die entstehenden Produkte selbst als Lösungsmittel für die Edukte fungieren können, kann zu Beginn des Verfahrens eine reduzierte Menge Lösungsmittel eingesetzt werden.

Die Reaktion kann diskontinuierlich in einem üblichen Druckbehälter (Autoklav) durchgeführt werden. Hierbei wird üblicherweise die Reaktionsmischung ständig innig vermengt. Beim Einsatz fester Katalysatoren ist bevorzugt, diese nach beendeter Reaktion sedimentieren zu lassen und die darüberstehende flüssige Phase abzusaugen oder abzulassen, um dann den Reaktor für den nächsten Ansatz mit frischen Edukten zu befüllen. Der Katalysator kann auf diese Weise ständig im Reaktor verbleiben.

Es ist bevorzugt, die Reaktion kontinuierlich durchzuführen. Hierzu ist ein üblicher Rohrreaktor geeignet. Dieser ist mit Vorteil so ausgelegt, daß er mit einer WHSV (weight hourly space velocity) von 1 bis 10 kg l⁻¹ h⁻¹ zu betreiben ist. Als optimal hat sich ein Bereich von 3 bis 8 kg l⁻¹ h⁻¹ erwiesen. Die Massenangabe in kg bezieht sich dabei auf die Gesamtmenge der eingesetzten Edukte.

Die nach dem erfindungsgemäßen Verfahren erhältlichen Polyolalkylether können als Lösungsmittel bzw. Lösungsvermittler, z. B. in Reinigungsmitteln, Farben und Lacken, eingesetzt werden. Diese Produkte weisen den Vorteil einer guten biologischen Abbaubarkeit auf.

Gegenstand der Erfindung ist desweiteren die Verwendung der Polyolalkylether als Additiv zur Verbesserung der Oktanzahl von Ottokraftstoffen. Die Polyolalkylether können Ottokraftstoffen in Mengen von 1 bis 25 V.A.-%, bezogen auf die Summe der Volumenanteile von Kraftstoff und Polyolalkylether, zugesetzt werden. Ein besonders günstiger Bereich liegt zwischen 5 bis 10 V.A.-%.

Bevorzugt wird das Reaktionsprodukt von Glycerin und Isobuten als Oktanzahlverbesserer in Ottokraftstoffen eingesetzt. Glycerin ist eine gut verfügbare Basischemikalie, die vorzugsweise aus nachwachsenden Rohstoffen, d. h. nativen Ölen und Fetten stammt. Größere Mengen Glycerin fallen z. B. bei der Verseifung von Fetten oder bei der Umesterung pflanzlicher Öle an. So kann z. B. Glycerin eingesetzt werden, das als Nebenprodukt bei der Umesterung von Rapsöl mit Methanol zu Rapsölmethylester (Bio-Diesel) entsteht.

Die Veretherung des Glycerins mit Olefinen der allg. Formel 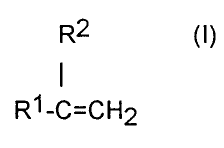
- R^{1:}: C₁-bis C₄-Alkylrest
- R^{2:}: C₁-bis C₄-Alkylrest oder H
z. B. Isobuten, ist mit dem erfindungsgemäßen Verfahren leicht durchführbar. Bei der Veretherungsreaktion entsteht ein Gemisch von 5 verschiedenen Ethern, die das Glycerin-Gerüst als Grundkörper enthalten. Bei der Veretherung mit Isobuten sind dies die 1-Mono-, 2-Mono-, 1,3-Di-, 1,2-Di- und Tri-tert.-butylether des Glycerins. Bereits die Monoether sind im Ottokraftstoff vollständig löslich.

Die Zusammensetzung des Gemisches läßt sich durch die Wahl des Verhältnisses der Edukte, z. B. durch das Verhältnis der jeweils eingesetzten Mole Glycerin zu Isobuten weitgehend beeinflussen und steuern. Ein Verhältnis 1 : 1 führt überwiegend zur Monosubstitution, während bei einem Verhältnis von 1 : 3 hohe Anteile des Triethers nachgewiesen werden können.

Bei höherem Olefin-Angebot können als Nebenprodukte auch das Dimere und Trimere des Olefins entstehen. Es ist jedoch nicht notwendig, diese Produkte nach erfolgter Reaktion aus dem Gemisch zu entfernen, wenn dieses zur Verbesserung der Oktanzahl Ottokraftstoffen zugesetzt werden soll. Der Glycerinumsatz und die Mengenverteilung der verschiedenen Ether sind im wesentlichen abhängig vom Olefin-Angebot.

Gemische mit einem hohen Anteil an Monoethern erzielen bei ROZ-Messungen die besten Blend-Oktanzahlen. Da bei der Herstellung von Gemischen mit einem hohen Monoetheranteil zwangsweise der Glycerin-Umsatz geringer ausfällt, wird das nicht umgesetzte Glycerin vorzugsweise aus dem Gemisch abgetrennt, um eine Phasentrennung bei der Mischung mit Benzin zu vermeiden, die eintreten würde, weil Glycerin nicht mit handelsüblichem Ottokraftstoff mischbar ist.

Ein bevorzugtes Lösungsmittel für die Veretherungsreaktion ist Methanol. Bei der Veretherung mit Isobuten entsteht dann als Nebenprodukt der Reaktion Methyltertiärbutylether (MTBE). In Bezug auf die oktanzahlverbessernde Wirkung des entstehenden Gemisches erweist sich diese Nebenreaktion als Vorteil, da bekanntermaßen Methyltertiärbutylether in großen Mengen als Oktanzahlverbesserer in Ottokraftstoffen eingesetzt wird. Ähnliches gilt auch für Ethanol als möglich einsetzbares Lösungsmittel, wobei dieses Ethanol auch aus nachwachsenden Rohstoffen stammen kann, z. B. als Gärungsalkohol.

Desweiteren kann mit Vorteil tert.-Butylalkohol (TBA) als Lösungsmittel für die Reaktion eingesetzt werden, da TBA ebenfalls ein Oktanzahlverbesserer ist. Vorteilhafterweise wird dem Reaktionsgemisch Wasser und eine entsprechend größere Menge Isobuten zugeführt, so daß das Lösungsmittel TBA unter den Reaktionsbedingungen des erfindungsgemäßen Verfahrens intermediär gebildet wird.

Nach beendeter Reaktion kann nicht umgesetztes Olefin z. B. Isobuten leicht über Kopf entfernt werden. Ggf. muß auch nicht umgesetztes Glycerin entfernt werden, z. B. durch Extraktion mit Wasser. Die verbleibende Mischung aus Ethern, Lösungsmittel und ggfs. Reaktionsprodukten der Nebenreaktion von Lösungsmittel mit Olefin kann direkt mit Ottokraftstoffen zur Erhöhung der Oktanzahl gemischt werden.

In den Fällen, in denen die entstehenden Produkte selbst als Lösungsmittel für die Edukte fungieren können, wird eine Ausführungsform des erfindungsgemäßen Verfahrens bevorzugt, bei dem das entstandene Rohprodukt fraktioniert wird und der Sumpf, der z. B. etwa 20 % des eingesetzten Rohprodukts ausmachen kann und der neben den Reaktionsprodukten auch nicht umgesetzte Polyhydroxyverbindungen, z. B. Glycerin enthält, wieder dem Verfahren zugeführt wird und als Lösungsmittel für frisch zugeführte Edukte dient. Diese Verfahrensweise hat den Vorteil, daß kein zusätzliches Lösungsmittel zugefügt werden muß, welches anschließend ggfs. wieder aus dem Rohprodukt entfernt werden muß und/oder mit den Edukten unerwünschte Nebenprodukte bildet. Diese Verfahrensvariante eignet sich insbesondere für die kontinuierliche Ausführungsform des erfindungsgemäßen Verfahrens.

Die Erfindung soll anhand der nachfolgenden Beispiele näher erläutert werden.

### Beispiele

### Herstellung von Glycerin-tert.-butylethern (GTBE)

### Beispiel 1 mit Methanol als Lösungsmittel

In einem Rührautoklaven (500 ml) wurden
46,5 g (0,5 Mol) Glycerin,
48,1 g (1,5 Mol) Methanol
174 g (3,1 Mol) i-Buten und
15 g Ionenaustauscher Amberlite® XE-386
unter Rühren (ca. 600 U/min) aufgeheizt. Bei 65 °C und einem Systemdruck von ca. 8 bar setzte die Veretherungsreaktion ein, erkennbar am Temperaturanstieg auf ca. 86 °C und dem langsam beginnenden Druckabfall auf 2 bar. Nach ca. 3 Stunden war die Reaktion beendet. Im Produktgemisch wurden durch GC folgende Komponenten nachgewiesen:

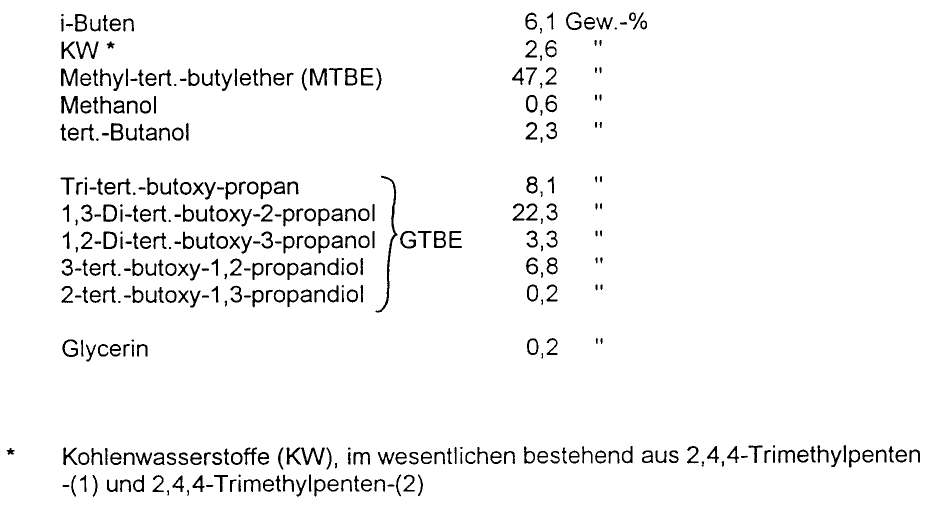

### Beispiel 2 mit Methanol als Lösungsmittel

Analog Bsp. 1 wurden
80 g (0,87 Mol) Glycerin
28 g (0,87 Mol) Methanol
196 g (3,49 Mol) i-Buten
eingesetzt.

Der Glycerinumsatz betrug > 99 %.

Nach Abtrennung des i-Butens und der Vorlauf-Kohlenwasserstoffe (KW) wurde folgendes Gemisch erhalten (GC-Analyse):

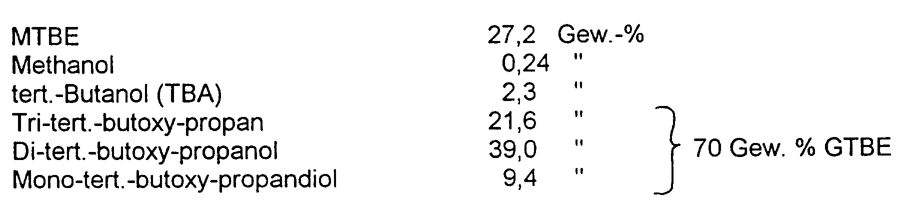

### Beispiel 3 mit tert.-Butanol als Lösungsmittel

Analog Bsp. 1 wurde folgendes Gemisch eingesetzt:
74 g (0,8 Mol) Glycerin
59,3 g (0,8 Mol) tert.-Butanol
180 g (3,2 Mol) i-Buten

Im Produktgemisch wurden folgende Komponenten nachgewiesen (GC):

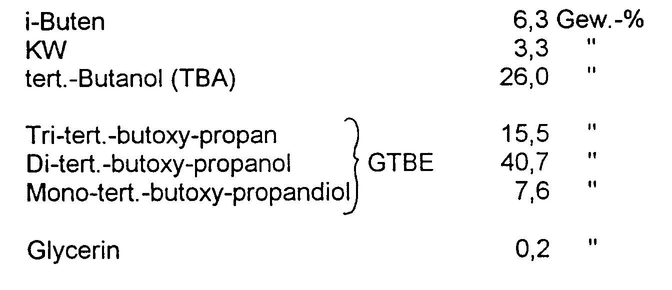

### Beispiel 3a mit tert.-Butanol als Lösungsmittel

95 g (0,95 Mol) Rohglycerin (Restwasser 4,9 %) und 52 g (0,70 Mol) tert.-Butanol wurden mit 4,5 g Methansulfonsäure vermischt und mit 214 g (3,8 Mol) i-Buten bei 90 °C und bei einem Druck von 20 bar im Autoklaven umgesetzt.

Nach der Neutralisation mit Natriumhydrogencarbonat und der Abtrennung des Vorlaufs wies das verbleibende Produktgemisch folgende Zusammensetzung auf (GC):

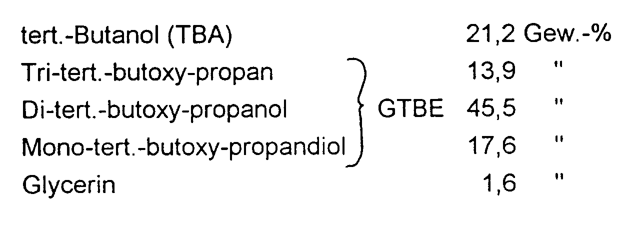

### Beispiel 4 mit Aceton als Lösungsmittel

Analog Bsp. 1 wurden
105 g (1,15 Mol) Glycerin
33,4 g (0,58 Mol) Aceton
194 g (3,46 Mol) i-Buten
eingesetzt.

Im Reaktionsprodukt betrug der Glyceringehalt < 0,05 Gew.-%.

Nach Abtrennung des Vorlaufs wurden im Reaktionsprodukt folgende Komponenten nachgewiesen (GC):

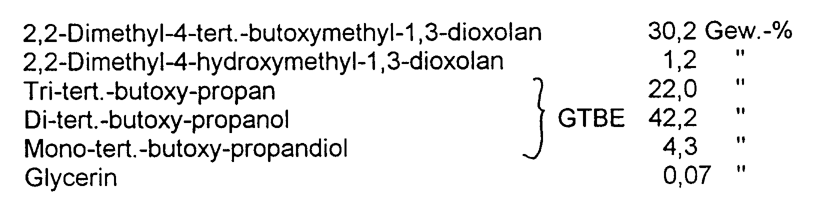

### Beispiel 5 mit Glycerinethern aus Bsp. 1 als Lösungsmittel

Analog zu Bsp. 1 wurden
92,1 g (1 Mol) Glycerin
50 g GTBE-Gemisch aus Bsp. 1
168 g (3 Mol) i-Buten
eingesetzt.

Die Reaktion wurde bei 90 °C durchgeführt. Der Anteil an Kohlenwasserstoffen (KW) betrug 40,2 Gew.-%.

Nach Destillation des Rohproduktes wurde folgende Zusammensetzung des GTBE-Gemisches ermittelt (GC):

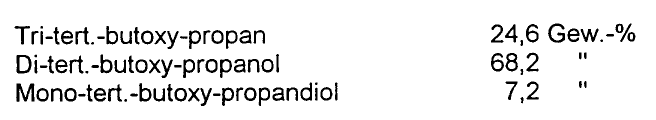

### Beispiele 6 bis 9

### Kontinuierliche Veretherung mit Methanol als Lösungsmittel

In einem Reaktionsrohr mit einer Länge von 750 mm, einem Durchmesser von 30 mm und gefüllt mit 170 g Ionenaustauscherharz Amberlite® XE-386 wurde Glycerin kontinuierlich mit i-Buten verethert.

Bei einer Belastung von WHSV = 4 kg l⁻¹ h⁻¹wurden kontinuierlich Glycerin, Methanol und i-Buten im Molverhältnis 1 : 3 : 6 in das Reaktionsrohr eingeführt. Der Druck wurde auf 25 bar geregelt. Eingangs- und Maximaltemperatur siehe Tabelle 1.

Aus der gaschromatischen Analyse des anfallenden Flüssigproduktes wurden die in der Tabelle 1 aufgeführten Ergebnisse ermittelt.

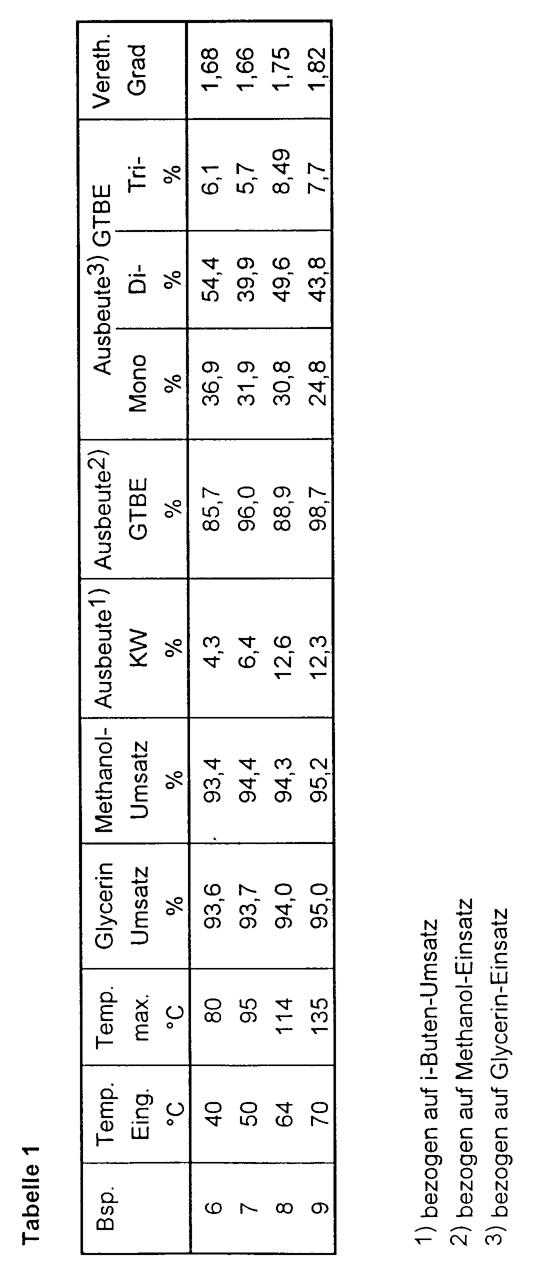

### Beispiel 10

### Kontinuierliche Veretherung mit Aceton als Lösungsmittel

Die kontinuierliche Glycerinveretherung mit i-Buten in Gegenwart von Aceton bzw. des Acetalgemisches aus Aceton und Glycerin wurde analog den Beispielen 6 bis 9 durchgeführt.

Das Molverhältnis betrug
Glycerin : Aceton : i-Buten = 1 : 1 : 4
WHSV: 4 kg l⁻¹ h⁻¹; Druck: 25 bar;
Eingangstemperatur: 62 °C; max. Temperatur: 85 °C

Nach Abtrennung von i-Buten, Aceton, KW und tert. Butanol als Vorlauf verblieb ein Reaktionsprodukt, welches laut GC-Analyse folgende Zusammensetzung aufwies:

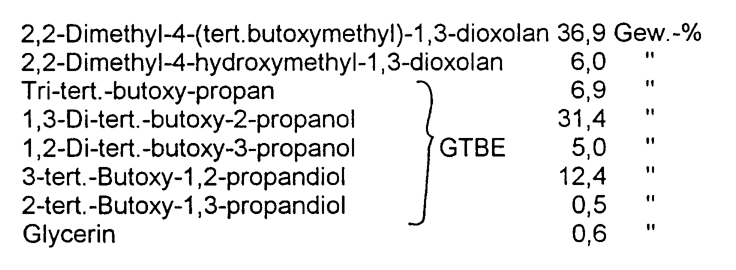

Das Gemisch ist als Kraftstoffkomponente im Ottokraftstoff einsetzbar (vgl. Tabelle 2).

**Tabelle 2**

| **Prüfung der Glycerinethermischungen als Kraftstoffkomponente** | | | | |
|---|---|---|---|---|
| | **ROZ** | **Blend-ROZ** | **MOZ** | **Blend- MOZ** |
| SOK | 97,1 | - | 85,7 | - |
| 90 VA | | | | |
| 10 VA GTBE/MTBE-Gemisch aus Bsp. 1 | 98,9 | 115 | 87 | 99 |
| 90 VA SOK | | | | |
| 10 VA GTBE/MTBE-Gemisch aus Bsp. 2 | 99 | 116 | 87 | 99 |
| 90 VA SOK | | | | |
| 10 VA GTBE/TBA-Gemisch aus Bsp. 3 | 98,6 | 112 | 86,8 | 95 |
| 90 VA | | | | |
| 10 VA GTBE/Dioxolanderivate aus Bsp. 4 | 98,4 | 110 | 86,5 | 94 |
| 90 VA SOK | | | | |
| 10 VA GTBE aus Bsp. 5 | 99,3 | 119 | 87 | 99 |
| 90 VA SOK | | | | |
| 10 VA GTBE/MTBE-Gemisch aus Mischung Bsp. 6 - 9 | 99,2 | 118 | 87,2 | 101 |
| 90 VA SOK | | | | |
| 10 VA Produktgemisch aus Bsp. 10 | 98,6 | 116 | 86,7 | 96 |
| SOK: Super-Ottokraftstoff VA: Volumen-Anteile ROZ: Research-Oktanzahl MOZ: Motor-Oktanzahl | | | | |

## Patentansprüche

1. Verfahren zur Herstellung von Polyolalkylethern, bei dem man Polyhydroxyverbindungen, ausgewählt aus der Gruppe
a) Alkylenglycole,
b) Glycerin,
d) Trimethylolpropan,
d) Pentaerythrit
in Gegenwart von sauren Katalysatoren bei Temperaturen von 50 bis 120°C und Drücken von 5 bis 30 bar mit Olefinen der Formel (I) 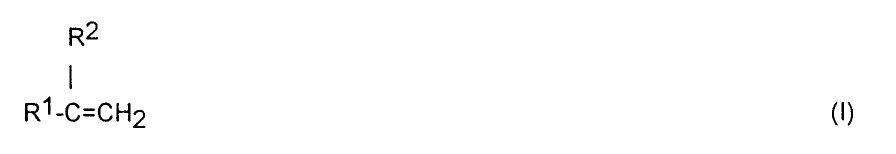 umsetzt, in der R¹ für einen linearen oder verzweigten Alkylrest mit 1 bis 4 Kohlenstoffatomen und R² für Wasserstoff oder ebenfalls für einen Alkylrest mit 1 bis 4 Kohlenstoffatomen steht, dadurch gekennzeichnet, daß man die Reaktion in der Flüssigphase in Gegenwart eines Lösungsmittels durchführt.

2. Verfahren Anspruch 1, dadurch gekennzeichnet, daß man als Lösungsmittel C₁- bis C₄-Alkohole, insbesondere Methanol und/oder - vorzugsweise intermediär gebildeten - tert.-Butylalkohol, einsetzt.

3. Verfahren nach Anspruch 1, dadurch gekennzeichnet, daß man als Lösungsmittel niedere Ketone, insbesondere mit 3 bis 9 C-Atomen, vorzugsweise Aceton, einsetzt.

4. Verfahren nach einem der vorstehenden Ansprüche, dadurch gekennzeichnet, daß man Polyhydroxyverbindungen und Lösungsmittel im Molverhältnis von 3 : 1 bis 1 : 5, insbesondere 2 : 1 bis 1 : 3, einsetzt.

5. Verfahren nach einem der vorstehenden Ansprüche, dadurch gekennzeichnet, daß man die Reaktion kontinuierlich in einem Rohrreaktor durchführt.

6. Verfahren nach Anspruch 5, dadurch gekennzeichnet, daß man den Rohrreaktor mit einer WHSV von 1 bis 10, insbesondere 3 bis 8 kg l⁻¹ h⁻¹, betreibt.

7. Verfahren nach einem der vorstehenden Ansprüche, dadurch gekennzeichnet, daß man als Olefin Isobuten einsetzt.

8. Verfahren nach einem der vorstehenden Ansprüche, dadurch gekennzeichnet, daß man als Polyhydroxyverbindung Glycerin einsetzt.

9. Verfahren nach einem der vorstehenden Ansprüche, dadurch gekennzeichnet, daß man unlösliche saure Ionenaustauscher als Katalysatoren einsetzt.

10. Verfahren nach einem der vorstehenden Ansprüche, dadurch gekennzeichnet, daß man die Polyhydroxyverbindungen und die Olefine im molaren Verhältnis von 1 : 1 bis 1 : 10 einsetzt.

11. Verwendung der Polyolalkylether als Oktanzahlverbesserer in Ottokraffstoffen.

12. Verwendung nach Anspruch 11, dadurch gekennzeichnet, daß die Polyolalkylether im Ottokraftstoff in Mengen von 1 bis 25, insbesondere 5 bis 10 V.A.-% eingesetzt werden.
